# EUROPEAN PATENT SPECIFICATION

(11) **EP 1 865 990 B1**
(45) Date of publication and mention of the grant of the patent: **03.11.2010**
(21) Application number: 06724633.0
(22) Date of filing: 07.04.2006
(51) Int. Cl.: A61K 47/10, A61K 47/12, A61K 31/565, A61P 5/50

(54) **METHOD OF TREATING TYPE-2 DIABETES**
VERFAHREN ZUR BEHANDLUNG VON DIABETES TYP 2
METHODE DE TRAITEMENT DU DIABETE DE TYPE 2

(30) Priority: 08.04.2005 US 669606 P
(43) Date of publication of application: 19.12.2007
(73) Proprietor: LABORATOIRES BESINS INTERNATIONAL, 75003 Paris (FR); Unimed Pharmaceuticals, LLC, Marietta, GA 30062 (US)
(72) Inventor: WOUN, Seo, Marietta, Georgia 30062 (US)
(74) Representative: Niemann, Frédéric
(86) International application number: PCT/EP2006/003974
(87) International publication number: WO 2006/108719

(56) References cited:
- US-A1- 2003 022 877
- US-B1- 6 503 894
- MÜLLER ET AL.: "Testosterontherapie des Hypogonadismus" SCHWEIZERISCHE ÄRZTEZEITUNG, vol. 81, no. 46, 2000, pages 2589-2593, XP002389108
- SHAPIRO J ET AL: "TESTOSTERONE AND OTHER ANABOLIC STEROIDS AS CARDIOVASCULAR DRUGS" AMERICAN JOURNAL OF THERAPEUTICS, CHAPMAN AND HALL, NEW YORK, NY, US, vol. 6, no. 3, May 1999 (1999-05), pages 167-174, XP009004530 ISSN: 1075-2765
- SIMON ET AL.: "Androgen Therapy Improves Insulin Sensivity and Decreases Leptin Level in Healthy Adult Men With Low Plasma Total Testosterone" DIABETES CARE, vol. 24, no. 12, December 2001 (2001-12), pages 2149-2151, XP002389109
- "Long-Term Testosterone Gel (AndroGel) Treatment Maintains Benefiial Effects on Sexual Function and Mood, Lean, and Fat Mass, and Bone Mineral Density in Hypogonadal Men" J. CLIN. ENDOCRINOL. METAB., vol. 85, no. 5, May 2004 (2004-05), pages 2085-2098, XP002389110
- Mutschler: "Arzneimittelwirkungen" Wissenschaftliche Verlagsgesellschaft , pages 405-418
- Wikipedia: "Glycated hemoglobin"
- UK PROSPECTIVE DIABETES STUDY GROUP: "Effect of intensive blood-glucose control with metformin on complications in overweight patients with type 2 diabetes (UKPDS 34)" THE LANCET, vol. 352, 12 September 1998 (1998-09-12), pages 854-865,
- CORRALES ET AL.: "Partial androgen deficiency in aging type 2 diabetic men and its relationship to glycemic control" METABOLISM, vol. 53, no. 5, 1 May 2004 (2004-05-01),

## Description

### FIELD OF THE INVENTION

The present invention is generally related to a transdermal hydroalcoholic gel composition for use in the treatment of type-2 diabetes in a subject in need thereof.

### BACKGROUND OF THE INVENTION

Type-2 diabetes is a carbohydrate metabolism disorder thought to be caused by a combination of hereditary and environmental factors. Individuals afflicted with type-2 diabetes typically demonstrate inadequate secretion or utilization of insulin, excessive urine production, and excessive amounts of sugar in the blood and urine. Established risk factors for the development of type-2 diabetes include obesity, an unfavorable body fat distribution, impaired glucose tolerance, hyperinsulinemia and insulin resistance. Insulin resistance, at least initially and often throughout the patient's lifetime, fundamentally underlies the pathophysiology of type-2 diabetes and improving insulin sensitivity is one of the primary therapeutic approaches and provides a valuable assessment of this disease state. Obesity, especially visceral obesity, and dyslipidemia have been reported to be associated with most of the type-2 diabetic subjects. They arc also the risk factors for developing the disease. One of the treatment goals in diabetes is to prevent chronic complications, which includes aggressive control of obesity, dyslipidemia and hypertension.

Mates suffering from type-2 diabetes have been shown to have lower testosterone levels than healthy men. Barrett-Connor, E., et al., Am. J. Epidemiol., 132(5):893-901 (1990). Type-2 diabetes often surfaces during middle-age, at the same time as male testosterone levels begin to decrease with age (andropause). Erectile dysfunction is a common complication of type-2 diabetes which often can be an early symptom and may cause depression.

Testosterone, the major circulating androgen in men, is synthesized from cholesterol. The approximately 500 million Leydig cells in the testers secrete more than 95% of the 6-7 mg of testosterone produced per day. Two hormones produced by the pituitary gland, luteinizing hormone ("LH") and follicle stimulating hormone ("FSH"), are required for the development and maintenance of testicular function and negatively regulate testosterone production. Circulating testosterone is metabolized to various 17-keto steroids through two different pathways. Testosterone can be metabolized to dihydrotestosterone ("DHT") by the enzyme 5α-reductase or to estradiol ("E2") by an aromatase enzyme complex.

Testosterone circulates in the blood 98% bound to protein. In men, approximately 40% of the binding is to the high-affinity sex hormone binding globulin ("SHBG"). The remaining 60% is bound weakly to albumin. Thus, a number of measurements for testosterone are available from clinical laboratories. The term "free" testosterone as used herein refers to the fraction of testosterone in the blood that is not bound to protein. The term "total testosterone" or "testosterone" as used herein means the free testosterone plus protein-bound testosterone. The term "bioavailable testosterone" as used herein refers to she non-SHBO bound testosterone and includes testosterone weakly bound to albumin.

The following table from the UCLA-Harbor Medical Center summarizes the hormone concentrations in normal adult men range:

**Table 1: Hormone Levels in Normal Men**

| **Hormone** | **Normal Range** |
|---|---|
| Testosterone | 298 to 1043 ng/dL |
| Free testosterone | 3.5 to 17.9 ng/dL |
| DHT | 31 to 193 ng/dL |
| DHT/T Ratio | 0.052 to 0.33 |
| DHT + T | 372 to 1349 ng/dL |
| SHBG | 10.8 to 46.6 nmol/L |
| FSH | 1.0 to 6.9 mlU/mL |
| LH | 1.0 to 8.1 mlU/mL |
| E₂ | 17.1 to 46.1 pg/mL |

There is considerable variation in the half-life of testosterone reported in the literature, ranging from 10 to 100 minutes. Researchers do agree, however, that circulating testosterone has a diurnal variation in normal young men. Maximum levels occur at approximately 6:00 to 8:00 a.m. with levels declining throughout the day. Characteristic profiles have a maximum testosterone level of 720 ng/dL and a minimum level of 430 ng/dL. The physiological significance of this diurnal cycle, if any, however, is not clear.

Mate hypogonadism results from a variety of patho-physiological conditions in which testosterone concentration is diminished below the normal range. The hypogonadic condition is sometimes linked with a number of physiological changes, such as diminished interest in sex, impotence, reduced lean body mass, decreased hone density, lowered mood, and decreased energy levels.

Researchers generally classify hypogonadism into one of three types. Primary hypogonadism includes the testicular failure due to congenital or acquired anorchia, XYY Syndrome, XX mates. Noonan's Syndrome, gonadal dysgenesis, Leydig cell tumors, maldescended testes, varicocele, Sertoli-Cell-Only Syndrome, cryptorchidism, bilateral torsion, vanishing testis syndrome, orchiectomy, Klinefelter's Syndrome, chemotherapy, toxic damage from alcohol or heavy mentals, and general disease (renal failure, liver cirrhosis, diabetes, myotonia dystrophica). Patients with primary hypogonadism show an intact feedback mechanism in that the low serum testosterone concentrations are associated with high FSH and LH concentrations. However, because of testicular or other failures, the high LH concentrations are not effective at stimulating testosterone production.

Secondary hypogonadism involves an idiopathic gonadotropin or LH-releasing hormone deficiency. This type of hypogonadism includes Kallman's Syndrome, Prader-Labhart-Willi's Syndrome. Laurence-Moon-Biedl's Syndrome, pituitary insufficiency/adenomas. Pasqualinl's Syndrome, hemochromatosis, hyperprolactinemia, or pituitary-hypothalamic injury from tumors, trauma, radiation, or obesity. Because patients with secondary hypogonadism do not demonstrate an intact feedback pathway, the lower testosterone concentrations are not associated with increased LH or FSH levels. Thus, these men have low testosterone serum levels but have gonadotropins in the normal to low range.

Third, hypogonadism may be age-related. Men experience a slow but continuous decline in average serum testosterone after approximately age 20 to 30 years. Researchers estimate that the decline is about 1-2% per year. Cross-sectional studies in men have found that the mean testosterone value at age 80 years is approximately 75% of that at age 30 years. Because the serum concentration of SHBG increases as men age, the fall in bioavailable and free testosterone is even greater than the fall in total testosterone. Researchers have estimated that approximately 50% of healthy men between the ages of 50 and 70 have levels of bioavailable testosterone that are below the lower normal limit. Moreover, as men age, the circadian rhythm of testosterone concentration is often muted, dampened, or completely lost. The major problem with aging appears to be within the hypothalamic-pituitary unit. For example, researchers have found that with ageing. LH levels do not increase despite the low testosterone levels, Regardless of the cause, these untreated testosterone deficiencies in older men may lead to a variety of physiological changes, including sexual dysfunction, decreased libido, loss of muscle mass, decreased hone density, depressed mood, and decreased cognitive function. The net result is geriatric hypogonadism, or what is commonly referred to as "male menopause." Today, hypogonadism is the most common hormone deficiency in men, affecting 5 in every 1,000 men. At present, it is estimated that only live percent of the estimated four to five million American men of all ages with hypogonadism currently receive testosterone replacement therapy.

Thus, there is a need in the art for a safe and effective treatment for treating, preventing, or reducing the risk of developing diabetes and for increasing glycemic control.

### SUMMARY OF THE INVENTION

The present invention is generally related to a transdermal hydroalcoholic gel composition for use in the treatment of type-2 diabetes in a subject in need thereof.

### DETAILED DESCRIPTION OF THE INVENTION

The present invention relates a transdermal hydroalcoholic gel composition for use in the treatment of type-2 diabetes in a subject in need thereof, wherein the transdermal hydroalcoholic gel composition comprises:
a. 0.01% to 15% (w/w) of testosterone;
b. 0.01% to 50% (w/w) penetration enhancing agent;
c. 0.01% to 50% (w/w) thickening agent;
d. 30% to 98% (w/w) lower alcohol; and
e. the balance water;
wherein the serum testosterone concentration in the subject is at least 600 ng/dl to 1050 ng/dl after at least 30 days of daily administration of the composition.

In one embodiment, the present invention is directed to the use of a hydroalcohoalic gel composition in the manufacture of a percutaneously deliverable medicament for treating type-2 diabetes and/or for increasing glycemic control in a subject in need thereof. The get comprises one or more lower alcohols, such as ethanol or isopropanol; a penetration enhancing agent: a thickener; and waster. Additionally, the present invention may optionally include salts, emollients, stabilizers, antimicrobials, fragrances, and propellants.

The present invention also includes kits, methods, combinations, and pharmaceutical compositions for treating, preventing, reversing, halting or slowing the progression of diabetes in a subject once it becomes clinically evident, or treating the symptoms associated with, or related to the diabetes. The subject may already have a diagnosis of diabetes at the time of administration, or be at risk of developing diabetes. The present invention further includes kits, methods, combinations, and pharmaceutical compositions for increasing glycemic control in a subject in need there of.

The term "derivative" refers to a compound that is produced from another compound of similar structure by the replacement of substitution of one atom, molecule or group by another. For example, a hydrogen atom of a compound may be substituted by alkyl, acyl, amino, etc., to produce a derivative of that compound.

As used herein, the term "lower alcohol," alone or in combination, means a straight-chain or branched-chain alcohol moiety containing one to about six carbon atoms. In one embodiment, the lower alcohol contains one to about 4 carbon atoms, and in another embodiment the lower alcohol contains two to about 3 carbon atoms. Examples of such alcohol moieties include methanol, ethanol, n-propanol, isopropanol, n-butanol, isobutanol, sec-butanol, and tert-butanol.

As used herein, the therm "lower alkyl", alone or in combination, means a straight-chain or branched chain alkyl radical containing one to about six carbon atoms, In one embodiment, the lower alkyl contains one to about four carbon atoms. Examples of such radicals include methyl, ethyl, n-propyl, isopropyl, n-butyl, isobutyl, sec-butyl, and tert-butyl.

The phrase "penetration enhancing agent" refers to an agent that accelerates the delivery of the drug through the skin. These agents also are referred to as accelerants, adjutants, and absorption promoters, and are collectively referred to herein as "enhancers." This class of agents includes those with diverse mechanisms of action including those which have the function of improving the solubility and diffusibility of the drug, and those which improve percutaneous absorption by changing the ability of the stratum corneum to retain moisture, softening the skin, improving the skin's permeability, acting as penetration assistants or hair-follicle openers or changing the state of the skin such as the boundary layer. The penetration enhancing agent of the present invention is a functional derivative of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the -COOH group is substituted with a functional derivative thereof, such as alcohols, polyols, amides and substituted derivatives thereof. The term "fatty acid" means a fatty acid that has four (4) to twenty-four (24) carbon atoms.

The composition is used in a "pharmacologically effective amount." This means that the concentration of the drug administered is such that in the composition it results in a therapeutic level of drug delivered over the term that the drug is to be used. Such delivery is dependent on a number of variables including the time period for which the individual dosage unit is to be used, the flux rate of the drug from the composition, for example, testosterone, from the gel, surface area of application site, etc. For testosterone, for example, the amount of testosterone necessary can be experimentally determined bused on the flux rate of testosterone through the gel, and through the skin when used with and without enhancers.

The term "prodrug" refers to a drug or compound in which the pharmacological action (active curative agent) results from conversion by metabolic processes within the body. Prodrugs are generally considered drug precursors that, following administration to a subject and subsequent absorption, are converted to an active or a more active species via some process, such as a metabolic process. Other products from the conversion process are easily disposed of by the body. Prodrugs generally have a chemical group present on the prodrug which renders it less active and/or confers solubility or some other property to the drug. Once the chemical group has been cleaved from the prodrug the more active drug is generated. Prodrugs may he designed us reversible drug derivatives and utilized as modifiers to enhance drug transport to site-specific tissues. The design of prodrugs to date has been to increase the effective water solubility of the therapeutic compound for targeting to regions where water is the principal solvent. For example. Fedorak, et al., Am. J. Physiol, 269:G210-218 (1995), describe dexamethasone- beta -D-glucuronide. McLoed, et al., Gastroenterol., 106:405-413 (1994), describe dexamethasone-succinate-dextrans. Hochhaus, et al., Biomed. Chrom., 6:283-286 (1992), describe dexamethasone-21-sulphobenzoate sodium and dexamethasone-21-isonicotinate. Additionally, J. Larsen and H. Bandgaard [Int. J. Pharmaceutics, 37, 87 (1987)] describe the evaluation of N-acylsufonamides as potential prodrug derivatives. J. Larsen et al., [Int. J. Pharmaceutics, 47, 103 (1988)] describe the evaluation of N-methylsulfonamides as potential prodrug derivatives. Prodrugs are also described in, for example, Sinkula et al., J. Pharm. Sci., 64:181-210 (1975). Other nonlimiting examples of "prodrugs" that can be used in the combinations and methods of the present invention include parecoxib (propanamide, N-[[4-(5-methyl-3-phenyl-4-isoxazolyl)phenyl]sulfonyl]-), and MAG-camptothecin.

In one embodiment, the present invention is directed to a method for percutaneous administration of testosterone in a hydroalcoholic gel. The gel comprises one or more lower alcohols, such as ethanol or isopropanol; a penetration enhancing agent; a thickener; and water. In one embodiment, the gel further comprises a hydroxide releasing agent, such as, e.g, sodium hydroxide. Additionally, the present invention may optionally include salts, emollients, stabilizers, antimicrobials, fragrances, and propellants.

Non-limiting examples of penetration enhancing agents include C8-C22 fatty acids such as isostearic acid, octanoic acid, and oleic acid; C8-C22 fatty alcohols such as oleyl alcohol and lauryl alcohol; lower alkyl esters or C8-C22 fatty acids such as ethyl oleate, isopropyl myristate, butyl stearate, and methyl laurate; di(lower)alkyl esters of C6-C22 diacids such as diisopropyl adipate; monoglycerides of C8-C22 fatty acids such as glyceryl monolaurate; tetrahydrofurfuryl alcohol polyethylene glycol ether; polyethylene glycol, propylene glycol,; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; N-alkylpyrrolidone; and terpenes.

The thickening agents (aka gelling agents) used herein may include anionic polymers such as polyacrylic acid (CARBOPOL® by B.F. Goodrich Specialty Polymers and Chemicals Division of Cleveland, Ohio); carboxypolymethylene, carboxymethylcellulose and the like, including derivatives of Carbopol®) polymers, such as Carbopol® Ultrez 10, Carbopol® 940, Carbopol® 941, Carbopol® 954, Carbopol® 980, Carbopol® 981, Carbopol® ETD 2001, Carbopol® EZ-2 and Carbopol® EZ-3, and other polymers such as Pemulen® polymeric emulsifiers, and Noveon® polycarbophils. Additional thickening agents, enhancers and adjuvants may generally be found in Remington's The Science and Practice of Pharmacy, Meade Publishing Co., United States Pharmacopeia/National Formulary.

In one embodiment, the formulation of the present invention delivers about 0.5 mg to about 250 mg testosterone, or the equivalent thereof, to a subject per dosage unit. In another embodiment of the present invention, the formulation delivers from about 5 mg to about 150 mg testosterone, or the equivalent thereof, to a subject per dosage unit. In yet another embodiment of the present invention, the formulations of the present invention deliver from about 25 mg to about 100 mg testosterone, or the equivalent thereof, to a subject per dosage unit. In another embodiment of the present invention, the formulations of the present invention deliver about 50 mg to about 100 mg testosterone, or the equivalent thereof, to a subject per dosage unit. In still another embodiment of the present invention, the formulations of the present invention deliver about 100 mg testosterone, or the equivalent thereof, to a subject per dosage unit. Thus, for example, a testosterone gel, ointment, cream or patch formulated for once a day administration can contain about 25 mg, or about 50 mg, or about 75 mg, or about 100 mg testosterone.

The formulation is a gel and is comprised of testosterone; a penetration enhancing agent, such as isopropyl myristate; a thickening agent, such as Carbopol; a lower alcohol, such as ethanol or isopropanol; and water. The formulation is a gel and is comprised of the following substances in approximate percentages:

**Table 2: Composition of Testosterone Formulation**

| **SUBSTANCE** | **AMOUNT (w/w)** |
|---|---|
| Testosterone | 0.01 - 15% |
| Penetration enhancing agent | 0.01 - 50% |
| Gelling agent | 0.01 - 50% |
| Lower alcohol | 30 - 98% |
| Purified water (qs) | to 100% |

In one embodiment, in a 100 g composition, the gel, ointment, cream, or patch may contain about 0.01 g to about 15 g of testosterone, about 0.01 g to about 50 g penetration enhancing agent, about 0.1 g to about 50 g gelling agent, and about 30 g to about 98 g lower alcohol. In another embodiment, in a 100 g composition, the gel, ointment, cream, or patch may contain about 0.1 g to 10 g of testosterone, about 0.1 g to about 5 g of penetration enhancing agent, about 0.1 g to about 5 g of gelling agent, an about 45 g to about 90 g lower alcohol and the balance water.

In one embodiment, the composition is a gel that further comprises sodium hydroxide or triethanolamine or potassium hydroxide, or a combination thereof, in an amount sufficient, as is known in the art, to assist the gelling agent in forming a gel. In one embodiment, a solution of sodium hydroxide is used, such as, e.g., 0.1 N sodium hydroxide solution, 0.2 N sodium hydroxide solution, 0.5 N sodium hydroxide solution, 1.0 N sodium hydroxide solution, 1.5 N sodium hydroxide solution, 2.0 N sodium hydroxide solution, or any other suitable solution for providing an amount sufficient of the sodium hydroxide to the composition. In one embodiment, the composition comprises about 1% to about 10% 0.1 N sodium hydroxide.

In another embodiment, the pharmaceutical composition includes about 0.5% to about 10% testosterone; about 30% to about 98% alcohol, for example, ethanol or isopropanol; about 0.1 % to about 5% isopropyl myristate; and about 0.1% to about 5% of a getting agent and the balance water. The percentages of components are weight to weight of the composition, In one embodiment, the composition comprises about 1% to about 10% 0.1 N sodium hydroxide.

The pharmaceutical composition includes testosterone in a hydroalcoholic gel. The testosterone may be present in a concentration of about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, about 7%, about 7.1%, about 7.2%, about 7.3%, about 7.4%, about 7.5%, about 7.6%, about 7.7%, about 7.8%, about 7.9%, about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, about 10.1%, about 10.2%, about 10.3%, about 10.4%, about 10.5%, about 10.6%, about 10.7%, about 10.8%, about 10.9%, about 11%, about 11.1%, about 11.2%, about 11.3%, about 11.4%, about 11.5%, about 11.6%, about 11.7%, shout 11.8%, about 11.9%, about 1.2%, about 12.1%, about 12.2%, about 12.3%, about 12.4%, about 12.5%, about 12.6%, about 12.7%, about 12.8%, about 12.9%, about 13%, about 13.1%, about 13.2%, about 13.3%, about 13.4%, about 13.5%, about 13.6%, about 13.7%, about 13.8%, about 13.9%, about 14%, about 14.1%, about 14.2%, about 14.3%, about 14.4%, about 14.5%, about 14.6%, about 14.7%, about 14.8%, about 14.9%, or about 15% weight to weight of the composition. The enhancer in this embodiment includes isopropyl myristate, which may be present in a concentration of about 0.5%, about 0.65%, about 0.75%, about 0.85%, about 0.95%, about 1%, about 2%, about 3%, about 4%, or about 5% weight to weight of the composition. The pharmaceutical composition also includes a C1-C4 alcohol present in a concentration of about 70%, about 71%, about 71.4%, about 71.8%, about 72%, about 72.3%, about 72.5%, about 72.7%, about 73%, about 73.5%, about 74%, about 74.5%, about 75% or about 75% weight to weight of the composition. Further, the pharmaceutical composition includes polyacrylic acid and/or carboxymethylcellulose as the gelling agent. In one embodiment, the gelling agent is polyacrylic acid present in a concentration of about 1% weight to weight of the composition.

One such testosterone gel has only recently been made available in the United States under the trademark AndroGel® by Unimed Pharmaceuticals, Inc., Marietta, Georgia, the assignee of this application. In one embodiment, the gel is comprised of the following substances in approximate amounts:

**Table 3: Composition of AndroGel^{®}**

| **SUBSTANCE** | **AMOUNT (w/w) PER 100g OF GEL** |
|---|---|
| Testosterone | 1.0 g |
| Carbopol 980 | 0.90 g |
| Isopropyl myristate | 0.50 g |
| 0.1 N NaOH | 4.72 g |
| Ethanol (96% v/v) | 71.4 g* |
| Purified water (gs) | to 100 g |

| | |
|---|---|
| *Corresponding to 67 g of ethanol | |

One skilled in the art will appreciate that the constituents of this formulation may be varied in amounts yet continue to be within the spirit and scope of the present intention. For example, the composition may contain about 0.1 to abort 10.0 g of testosterone, about 0.1 to about 5.4 g CARBOPOL, about 0.1 to about 5.0 g isopropyl myristate, and about 30.0 to about 98.0 g ethanol.

In still another embodiment, the composition comprises testosterone in an amount greater than 0.01%, a penetration enhancing agent in an amount greater than about 0.1%, a thickening agent in an amount greater than about 0.1%, sand a lower alcohol in an amount greater than about 30% w/w of the composition.

The gel is rubbed or placed onto an area of skin of the subject and allowed to dry. Illustratively, the gel is rubbed onto an area of skin, for example, on the upper outer thigh and/or hip once daily. Following application the subject washes his or her hands. Application of the gel results in an increased testosterone level having a desirable pharmacokinetic profile and is effective to treat or prevent diabetes, or the symptoms associated with, or related to diabetes, or to increase glycemic control in the subject. The composition is thus useful for treating a number of conditions or diseases in both men and women.

In one embodiment, the present invention employs a packet having a polyethylene liner compatible with the components of a testosterone gel, as described below. The packet may hold a unit dose or multiple dose.

In another embodiment, the methods and compositions employ a composition that is dispensed from u rigid multi-dose container (for example, with a hand pump) having a larger foil packet, for example, of the composition inside the container. Such larger packets can also comprise a polyethylene liner as above. In one embodiment, the multi-dose container comprises an airless pump that comprises a polyethylene pouch within a canister with a hand pump inserted. In one embodiment, the polyethylene pouch comprises 44 g or 88 g of product. In one embodiment, the pump is primed before use, such as, e.g., by fully depressing the pump three times and discarding the gel. In one embodiment, the pump contains enough product to allow for priming and a set number of precise doses. In one embodiment, each full pump depression delivers 1.25 g of testosterone gel. In this embodiment, a 3.75 g dose of gel would require 3 pump depression. A 5 g dose of gel would require 4 pump depressions. A 7.5 g dose of gel would require 6 pump depressions. A 10 g dose of gel would require 8 depressions, and so on. Of course, each pump depression can deliver any amount of testosterone gel suitable for delivering the desired dose.

It has been shown, and is discussed in U.S. Patent No. 6,503,894, U.S. Published Patent Applications 2002/0183290, 2003/0022877, 2003/0050292, 20031/0139384, 2003/0232072, 2004/0002482, 2004/0092494, and U.S. Patent Applications Ser. Nos. 09/703,753, 10/787,071, 10/825,540, 10/828,678, 10/829,618, 10/867,435, 10/924,421, and 10/925,421, herein incorporated by reference in their entirety, that transdermal application of testosterone using AndroGel® to hypogonadal men results in improved testosterone levels, mood, libido and sexual performance. As disclosed herein, it has now been discovered that AndroGel® may also be used for the treatment or prevention of diabetes, or for the increase in glycemic control in a subject.

The methods and compositions of the present invention provide enhanced treatment options for treating diabetes in a subject, for example, a man, as compared to those currently available. The methods and compositions of the present invention provide enhanced treatment options for increasing glycemic control in a subject, for example, a man, as compared to those currently available.

In one embodiment, the pharmaceutical composition of the present invention is administered once, twice, or three times a day, or as many times necessary to achieve the desired therapeutic effect. In another embodiment the composition of the present invention is administered once, twice, or three times a day on alternate days. In another embodiment the composition of the present invention is administered once, twice, or three times a day on a weekly, biweekly, or monthly basis.

Besides being useful for human treatment, the present invention is also useful for veterinary treatment of mammals, reptiles, birds, exotic animals and farm animals, including mammals, rodents, and the like. In one embodiment, the mammal includes a primate, for example, a human, a monkey, or a lemur, a horse, a dog, a pig, or a cat. In another embodiment, the rodent includes a rat, a mouse, a squirrel or a guinea pig,

In one embodiment of the present invention a method is provided for treating diabetes in a subject in need thereof, that is, a subject indicated for having, or at risk of developing diabetes. The method comprises administering a pharmacologically effective amount of a composition to an area of skin of the subject for delivery of testosterone to blood serum of the subject. The composition comprises: about 0.01% to about 15% (w/w) testosterone: about 0.01% to about 50% (w/w) penetration enhancing agent; about 0.01% to about 50% (w/w) gelling agent; about 30% to about 98% (w/w) lower alcohol; and the balance water.

The composition is capable of releasing the steroid after applying the composition to the skin at a rate and duration that delivers in one embodiment of the present invention at least about 10 µg per day of the steroid to the blood serum of the subject.

In another embodiment of the present invention, the composition is capable of releasing the testosterone after applying the composition to the skin of a subject at a rate and duration that achieves a circulating serum concentration of testosterone greater than about 400 ng per dl scrum during a time period beginning about 2 hours after administration and ending about 24 hours after administration.

In another embodiment of the present invention, the composition is capable of releasing the testosterone after applying the composition to the skin of a subject at a rate and duration that achieves a circulating serum concentration of the testosterone between about 400 ng testosterone per dl serum to about 1050 ng testosterone per dl scrum.

In another embodiment of the present invention, for each about 0.1 gram per day application of the composition of the present invention to the skin of a subject, an increase of at least about 5 ng/dl in serum testosterone concentration results in the subject.

In another embodiment of the present invention, the composition of the present invention is provided to a subject for daily administration in about a 0.1 g to about a 10 g dose. The composition of the present invention can be provided in any suitable dose, such as, e.g., from about 0.1 g to about 10 g, for example, about 0.1 g, about 0.44 g, about 0.88 g, about 1 g, about 1.32 g, about 1.5 g, about 1.75 g, about 2 g, about 2.25 g, about 2.5 g. about 2.75 g, about 3 g, about 3.5 g, about 3.75 g, about 4 g, about 4.25 g, about 4.5 g, about 4.75 g, about 5 g, about 5.25 g, about 5.5 g, about 5.75 g, about 6 g, about 6.25 g, about 6.5 g, about 6.75 g, about 7 g, about 7.25 g, about 7.5 g, about 7.75 g, about 8 g, about 8.25 g, about 8.5 g, about 8.75 g, about 9 g, about 9.25 g, about 9.5 g, about 9.75 g, about 10 g, or any other suitable dose.

In one embodiment of the invention, a 3.75 g dose of the composition of the present invention contains 37.5 mg of testosterone, a 5 g dose of the composition of the present invention contains 50 mg of testosterone, a 7.5 g dose of the composition of the present invention contains 75 mg, and a 10 g dose of the composition of the present invention contains 100 mg of testosterone.

In yet another embodiment of the present invention, the subject in need of treatment has a serum total testosterone level before the first application (pretreatment) of the composition of the present invention of less than 300 ng/dl.

In the present invention, where after at least about 30 days of daily administration of the composition of the present invention the serum testosterone concentration in a subject is at least 600 ng/dl to 1050 ng/dl, or 700 ng/dl to 1050 ng/dl.

In still another embodiment of the present invention, where after daily administration of the composition of the present invention the total testosterone concentration in a subject is greater than 300 ng/dl. In one embodiment, the total serum androgen concentration in the subject is greater than 600 ng/dl or 700 ng/dl, In one embodiment, the total testosterone concentration is measured after 24 hours of administration. In one embodiment, the total testosterone concentration is measured after 2 days of daily administration, such as, for example, after 10 days, 20 days, or 30 days.

In another embodiment of the methods, kits, combinations, and compositions of the present invention, the composition of the present invention is administered once, twice, or three times daily to a subject for at least about 7 days. In one embodiment, the composition is administered once a day.

The present invention provides a method of treating diabetes in a subject in need thereof, that is, to subject indicated for having diabetes, by administering to the subject an amount of a composition composing: about 0.5% to about 10% (w/w) testosterone: about 0.1% to about 5% (w/w) penetration enhancing agent; about 0.1% to about 5% (w/w) thickening agent; about 30% to about 98% (w/w) lower alcohol; and the balance water.

The present invention also provides a method for treating diabetes in a subject comprising: administering to a subject in need thereof an effective amount of a pharmaceutical composition comprising: about 0.1% to about 10% (w/w) testosterone: about 0.1% to about 5% (w/w) isopropyl myristate; about 0.1% to about 5% (w/w) thickening agent; and about 30% to about 98% (w/w) lower alcohol. In one embodiment, the thickening agent is polyacrylic acid, such as, Carbopol® and the composition further comprises a hydroxide releasing agents, such as, e.g., sodium hydroxide.

Achieving target delivery rates demonstrated by testosterone get can be estimated from the pharmacokinetics in testosterone gel in men. The mean scrum concentration (Cavg) values in men after applying of varying amounts of gel to the upper body is given in the Table below.

**Table 4: Mean Average Serum Testosterone Concentrations and Daily Delivery Rate after Administration of Testosterone Gel 1% In Men**

| Dose (µL) (gram) | Mean Cavg (ng/dL) | Daily Delivery Rate (µg/day)^{a} |
|---|---|---|
| 5.0 | 5.55 (± 225) | 3330 |
| 7.5 | 601 (± 309) | 3606 |
| 10 | 713 (± 209) | 4278 |

| | | |
|---|---|---|
| ^{a} Metabolic Clearance Rate of Daily Testosterone = 600 L/day | | |

Based on the results obtained in men, a testosterone gel dose of 0.5 grams delivers approximately 300 µg of testosterone per day.

The present invention is further illustrated by the following examples, which should not be construed as limiting in any way. The practice of the present invention will employ, unless otherwise indicated, conventional techniques of pharmacology and pharmaceutics, which are within the skill of the art.

### EXAMPLES

### Example 1: Prevalence of Hypogonadism in Men With Hypertension

This example demonstrates the prevalence of hypogonadism in men aged at least 45 years who present to primary care offices, regardless of the reason for the visit. To examine whether the occurrence of hypogonadism was associated with recognized components of the metabolic syndrome in this patient group, including hypertension, hyperlipidemia, and increased body mass.

### METHODS

**Study Design:** The study was a cross-sectional survey to determine the prevalence of hypogonadism in patients aged at least 45 years who were seen before noon in primary care offices during a 2-week period. Clinicians from a random sample of 2650 primary care practices throughout the United States were contacted. 130 practices qualified for participation. Men who were seen in a participating physician's office between 8 AM and noon during a 2-week period, regardless of the reason for their visit, were invited to participate in the study.

Inclusion criteria included: age 45 years or older, ability to provide a blood sample, willingness to answer a brief set of questions related to medical history, social history, concomitant medications, and hypogonadism-related signs and symptoms, and the ability to read, speak, and understand English, Exclusion criteria included the inability or unwillingness to sign the unformed consent form.

**Assessments:** All eligible patients had a single morning blood draw (between 8 AM and noon) to test for concentrations of total testosterone (TT), free testosterone (FT), bioavailable testosterone (BAT), and sex hormone-binding globulin (SHBG). All blood tests were analyzed by Esoterix Labs, Austin, TX.

Demographic characteristics, medical history, social history, and concomitant medications were collected to capture the following information: symptoms associated with hypogonadism, decline in general feeling of well-being, decrease in muscular strength/feeling of weakness, physical exhaustion/lacking vitality, decrease in sexual desire/libido, decrease in ability/frequency to perform sexually, depressed mood, and comorbid conditions.

**Statistical Analysis:** The primary analyses focused on descriptive statistics and prevalence estimation for hypogonadism, defined as TT <300 ng/dL. Prevalence estimates (with 95% confidence interval |CI|) of hypogonadism were also obtained for subgroups of patients derived from demographic variables and other underlying conditions (risk factors). A second exploratory analysis was conducted to assess the impact of demographic variables and identify potential risk factors that were associated with hypogonadism. Odds ratios and corresponding 95% CIs were determined for each factor in the analysis. The Hosmer-Lemeshow goodness-of-fit test was run on the final stepwise regression analysis model to check the model's adequacy for the data.

### RESULTS

Of the 2650 primary care practices throughout the United States contacted to participate, 95 practices enrolled patents (Family medicine 51%, Internal medicine 42%). Of the 2498 men who were solicited to participate, 2165 of them enrolled in the study (87% acceptance rate).

| **Table 1. Demographics of Enrolled Patients** | | | |
|---|---|---|---|
| | **Hypogonadal Patients** | **Eugonadal Patients** | **Total Patients*** |
| **Characteristic** | **(n=836)** | **(n=1326)** | **(N=2165)** |
| Race, n (%) | | | |
| White | 700 (83.7) | 1077 (81.2) | 1780 (82.2) |
| Black | 114 (13.6) | 180 (13.6) | 294 (13.6) |
| Hispanik | 15 (1.8) | 42 (3.2) | 57 (2.6) |
| Asian | 2 (0.2) | 11 (0.8) | 13 (0.6) |
| Other | 5 (0.6) | 16 (1.2) | 21 (1.0) |
| Mean age (± SD), y | 61.6 (10.57) | 59.9 (10.11) | 60.5 (10.33) |
| Mean BMI* (± SD), kg/m² | 31.5 (6.06) | 28.5 (5.04) | 29.7 (5.64) |
| BMI=body mass index; SD= standard deviation. | | | |
| *Evaluable total testosterone values were not available for 3 patients. | | | |

| **Table 2. Reason for Physician Office Visit for Enrolled Patients** | |
|---|---|
| **Reason for Visit, n (%)** | **Patients (n=2098)*** |
| General checkup | 1293 (61.6) |
| Cardiovascular | 249 (12.0) |
| Respiratory | 163 (8.0) |
| Skeletal | 137 (6.5) |
| Other | 251 (12.1) |
| *Total number of enrolled patients was 2165; however, the reason for the visit was not recorded for 67 patients. | |

Most men (61.6%) were visiting their physicians for routine care. Two hundred forty-nine men (12%) presented for cardiovascular-related visits.

Of 2162 patients enrolled in the study with evaluable testosterone levels, 836 (38.7%) were hypogonadal (TT <300 ng/dL or being treated for hypogonadism). 80 patients on current testosterone therapy were considered hypogonadal, regardless of TT value. In 2082 patients not receiving testosterone, 756 (36.3%) were hypogonadal (95% CI, 34.2%-38.4%). The mean TT concentration in all patients was 364.8 ng/dL. The crude prevalence of hypogonadism (based on TT) for all patients was 38.7%. Consistent with the comparison of TT between groups, when BAT, FT, and SHBG values were stratified by hypogonadal status, significant differences were observed between groups (*P*<0.001).

| **Table 3. Testoterone Levels Stratified by Hypogonadal Status** | | | |
|---|---|---|---|
| **Laboratory Test** | **Hypogonadal** | **Eugonadal** | |
| **(mean ± SEM)** | **(TT <300)** | **(TT ≧ 300)** | ***P* value** |
| TT (ng/dL) | 245.6±4.12 | 439.9±3.52 | 0.001 |
| | n=836 | n=1326 | |
| Bioavailable testosterone (ng/dL) | 86.1±2.4 | 108.8±1.3 | 0.001 |
| | n=821 | n=1317 | |
| Free testosterone (pg/mL) | 47.9±1.03 | 63.9±0.53 | 0.001 |
| | n=834 | n=1325 | |
| SHBG (nmol/L) | 43.7±0.74 | 68.3±0.87 | 0.001 |
| | n=836 | n=1326 | |
| SEM=standard error of the mean; SHBG=sex hormone-binding globulin; TT=total testosterone. | | | |

The prevalence of hypogonadism in untreated hypogonadal patients (n=2085) was 36.3% (95% CI. 34.2%-38.4%).

| **Table 4. Medical History of Enrolled Patients With Evaluable Total Testosterone** | | | |
|---|---|---|---|
| | **Hypogonadal Patients** | **Eugonadal Patients** | |
| **Condition, n(%)** | **(n=836)** | **(n=1326)** | ***P* Value*** |
| Hypertension | 547 (65.4) | 678 (51.1) | <0.001 |
| Hyperlipidemia | 506 (60.5) | 670 (50.5) | <0.001 |
| Diabetes | 258 (30.9) | 237 (17.9) | <0.001 |
| Obesity | 270 (32.3) | 225 (17.0) | <0.001 |
| Prostatic disease/disorder | 165 (19.7) | 226 (17.0) | 0.121 |
| Chronic pain | 155 (18.5) | 211 (16.0) | NS |
| Insomnia/sleep disturbance | 129 (15.4) | 185 (14.0) | NS |
| Asthma/COPD | 102 (12.2) | 118 (8.9) | NS |
| Headaches (within the last 2wk) | 70 (8.4) | 125 (9.4) | NS |
| Rheumatoid arthritis | 28 (3.3) | 29 (2.2) | NS |
| Osteoporosis | 15 (1.8) | 15 (1.1) | NS |
| Not reported | 0 (0.0) | 4 (0.3) | NS |
| COPD=chronoic obstructive pulmonary disease; NS=not significant. | | | |
| **P* values obtained franchi-square test of hypogonadal versus eugonadal patients. | | | |

A significantly higher proportion of hypogonadal than cugonadal patients reported a history of recognized components of the metabolic syndrome: hypertension, hyperlipidemia, diabetes, and obesity (*P*<0.001 for all of the conditions).

| **Table 5. Symptons of hypogonadism in Enrolled Patients With Hypertension** | | | |
|---|---|---|---|
| | **Hypogonadal Patients** | **Eugonadal Patients** | |
| **Signs and Symptoms, n(%)** | **(n=547)** | **(n=678)** | ***P* Value*** |
| Decrease in ability/frequency to perform sexually | 305 (55.8) | 330 (48.7) | 0.014 |
| Decrease in sexual desire/libido | 248 (45.3) | 286 (42.2) | 0.268 |
| Physical exhaustion/lacking vitality | 166 (30.3) | 189 (27.9) | 0.343 |
| Decrease in muscular strength (feeling of weakness) | 149 (27.2) | 171 (25.2) | 0.424 |
| Decline in general feeling of well-being | 138 (25.2) | 152 (22.4) | 0.250 |
| Depressive mood | 100 (18.3) | 118 (17.4) | 0.690 |
| **P* values obtained from chi-square test of hypogonadal versus eugonadal patients. | | | |

Decreased ability/frequency to perform sexually was the most common symptom of hypogonadism among these men, reported by 55.8% (*P*=0.014 vs eugonadal group). A significantly greater proportion of hypogonadal men versus eugonadal men with a history of diabetes, hypertension, or hyperlipidemia reported a decrease in ability/frequency to perform sexually (P ≤0.014). Decrease in sexual desire/libido and feelings of physical exhaustion/lacking vitality were significantly increased in hypogonadal versus eugonadal men with a history of hyperlipidemia or diabetes (P ≤0.023). A decline in general feeling of wellbeing was significantly more common in hypogonadal men with hyperlipidemia than in eugonadal men (P=0.011).

| **Table 6. Prevalence and Odds Ratios for hypogonadism in Untreated Patients With Hypertension and Components of the Metabolic Syndrome** | | | |
|---|---|---|---|
| | | **Hypogonadism** | **Odds Ratio** |
| **Risk Factor/Condition** | **n** | **Prevalence, %** | **(95% CI)** |
| Hypertension | 1177 | 42.4 | 1.84 (1.53-2.22) |
| Diabetes | 474 | 50.0 | 2.09 (1.70-2.58) |
| Hyperlipidemia | 1125 | 40.4 | 1.47 (1.23-1.76) |
| BMI ≥25 kg/m² | 1607 | 39.3 | 3.74 (2.07-3.07) |
| Age ≥65y | 684 | 39.9 | 1.26 (1.08-1.28) |
| BMI=body mass index; CI=confidence interval; untreated=not currently being treated for hypogonadism. | | | |

The prevalence of hypertension in untreated hypogonadal patients was 42.4%, and the odds of having hypogonadism were 1.84 times higher in men with a history of hypertension than in normotensive men.

| **Table 7. Summary of Odds Ratios of Hypogonadism From Stepwise Regression Analysis for Untreated Patients** | |
|---|---|
| **Risk Factor** | **Odds Ratio (95% CI)** |
| Age (10-y increase) | 1.21 (1.10-1.34) |
| BMI, kg/m² (5-unit increase) | 1.63 (1.48-1.80) |
| Diabetes | 1.37 (1.08-1.73) |
| Hypertension | 1.32 (1.07-1.63) |
| BMI=body mass index; CI=confidence interval. | |

The odds ratio obtained from stepwise regression analysis (used to examine the correlation among risk factors) confirmed the association of age, increased BMI, diabetes, and hypertension with hypogonadism.

The prevalence of hypogonadism in enrolled patients with diabetes is shown in Table 8.

**Table 8: Prevalence of Hypogonadism in Enrolled Patients With Diabetes**

| **Signs and Symptoms, n(%)** | **Hypogonadal patients (n=258)** | **Eugonadal patients (n=237)** | **P value*** |
|---|---|---|---|
| **Decrease in ability/frequency to perform sexually** | 169(65.5) | 125 (52.7) | 0.004 |
| **Decrease in sexual desire/libido** | 143 (55.4) | 98 (41.4) | 0.002 |
| **Physical exhaustion/lacking vitality** | 92 (35.7) | 62 (26.2) | 0.023 |
| **Decrease in muscular strength/feeling of weakness** | 81 (31.4) | 70 (29.5) | 0.654 |
| **Decline in general feeling of well-being** | 73 (28.3) | 60(25.3) | 0.455 |
| **Depressive mood** | 49(19.0) | 44(18.6) | 0.903 |

| | | | |
|---|---|---|---|
| *P values obtained from chi-square test testing hypogonadal vs. eugonadal patients | | | |

The prevalence of hypogonadism in enrolled patients with hyperlipidemia is shown in Table 9.

**Table 9: Prevalence of Hypogonadism in Enrolled Patients With Hyperlipidemin**

| **Signs and Symptoms, n(%)** | **Hypogonadal patients (n=506)** | **Eugonadal patients (n=670)** | **P value*** |
|---|---|---|---|
| **Decrease in ability/frequency to perform sexually** | 263 (52.0) | 276 (41.2) | <0.001 |
| **Decrease in sexual desire/libido** | 220 (43.5) | 244 (36.4) | 0.014 |
| **Physical exhaustion/lacking vitality** | 154 (30.4) | 156 (23.3) | 0.006 |
| **Decrease in muscular strength/feeling of weakness** | 130 (25.7) | 150 (22.4) | 0.188 |
| **Decline in general feeling of well-being** | 127 (25.1) | 127 (19.0) | 0.011 |
| **Depressive mood** | 92 (18.2) | 103 (15.4) | 0.200 |

| | | | |
|---|---|---|---|
| *P values obtained from chi-square test testing hypogonadal vs. eugonadal patients | | | |

### CONCLUSION

In this study, based on TT <300 ng/dL, the prevalence of hypogonadism among the men aged 45 years or older was estimated to be 38.7%. The prevalence of hypertension in untreated hypogonadal patients was 42.4%, and hypertensive men were 1.84 times more likely to have hypogonadism. The most common symptom of hypogonadism among these men was decreased ability/frequency to perform sexually, reported by 55.8% of hypogonadal men. The prevalence of hypogonadism in the HIM study increased with advancing age, which is consistent with findings from other studies. The relative risk of hypogonadism was greater with each 10-year increase in age. This example demonstrates that a significantly higher proportion of hypogonadal than eugonadal patients reported a history of hypertension and other recognized components of the metabolic syndrome: hyperlipidemia, diabetes, and increased body mass.

### Example 2: Effect of the Administration of 1% Testosterone Gel on Glycemic Control in Hypogonadal Men With Type 2 Diabetes

This example will demonstrate that percutaneous administration of testosterone gel results in an increase in the glycemic control (mean change in glycosylated hemoglobin (A1C) from baseline to Week 26) of hypogonadal type 2 diabetic males who have had moderate control (A1C, 7.0% to 9.0%) on a stable dosing regimen (842 weeks) of oral hypoglycemic agents.

Hypogonadal men aged 30 through 80 years with a diagnosis of type 2 diabetes, who have had moderate glycemic control (A1C. 7.0% to 9.0%) on a stable dosing regimen of oral hypoglycemic agents will be enrolled in a multi-center, double-blind, randomized, placebo-controlled, parallel group, dose-adjustment study. Subjects who consent to participate in the study must exhibit scrum total testosterone concentration of <300 ng/dL at the pre-screen visit and have a body mass index (BMI) of 25-40 kg/m2. Once these requirements are met and the remaining inclusion/exclusion criteria are fulfilled, subjects will enter the 8-week Screening Period. Hypogonadal subjects on a stable dosing regimen of oral hypoglycemic agents, remaining moderately controlled (A1C, 7.0% to 9.0%) and who exhibit serum total testosterone concentration of <300 ng/dL at the pre-screen visit will be selected for randomization. A total of 180 eligible subjects will be randomized at baseline in a 1:1 ratio to receive a 26-week treatment of 1% testosterone gel or matching placebo gel treatment.

The initial dose for the first 2 weeks after randomization will be 7.5 g of study medication (1% of testosterone gel or placebo) each day. This starting dose was selected to rapidly achieve the target range of morning serum total testosterone concentration of 600 ng/dL to 1000 ng/dL during the dose titration period. This target range represents high-mid to high-normal total testosterone levels. At the end of two weeks, serum testosterone concentrations will be determined. Subjects who do not achieve the target range of morning serum total testosterone concentration (600 ng/dL to 1000 ng/dL) will have their dose adjusted by 2.5 g every two weeks until Week 6 with a maximum dose of 15.0 g, or the target serum testosterone range is reached, whichever occurs earlier. Subjects will remain on this dose for the remainder of the study.

At any time during the study, if the serum total testosterone concentration is >1000 ng/dL, the dose will be decreased by 2.5 g every two weeks until the serum total testosterone concentration falls within the target range of 600 ng/dL to 1000 ng/dL, or a minimum dose of 5.0 g/day, whichever occurs earlier. In subjects with total serum testosterone levels >1000 ng/dL when receiving the minimum labeled dose of 5.0 g/day for at least 2 weeks, dose should be lowered to 2.5 g/day. Subjects should be discontinued if the total serum testosterone level is still >1000 ng/dL after 2 weeks at a dose of 2.5 g/day.

Total testosterone, free testosterone, bioavailable testosterone, SHBG, lutenizing hormone (LH) and estradiol will be collected and analyzed. A1C, fasting blood glucose, fasting blood insulin, C-peptide, Apo(a), leptin, fructosamine, and a lipid profile, including total cholesterol, LDL cholesterol, HDL cholesterol, non-HDL cholesterol, and triglycerides will also be measured. Subjects' body weight, body mass index (BMI), waist circumference, waist-to-hip ratio and skin fold thickness will be analyzed. A computed tomography (CT) scan will be used to determine visceral body fat. A dual energy x-ray absorptiometry (DEXA) scan will be used to determine lean body mass. The 17-GRID Hamilton Depression Rating Scale is a 17-item screening instrument designed to measure the severity of illness in adults already diagnosed as having depression and will be administered to patients throughout the study. The International index of Erectile Function (HEF) is a validated, multidimensional self-administered questionnaire that consists of 15 questions and is used to evaluate erectile dysfunction and treatment outcomes in clinical trials, and will also be administered to patients at visits during the study. Hypoglycemia incidents will be recorded. Hematology, blood chemistry, prostate-specific antigen (PSA), physical examination, digital rectal examination, international prostate symptom scale, and electrocardiogram reading will also be collected.

The intent-to-treat (ITT) population consists of all randomized subjects who administered at least one dose application of study medication, and have at least one post-baseline efficacy measurement. The per protocol population consists of all ITT subjects who did not violate the protocol in any substantial manner.

The primary efficacy parameter is defined to he the mean change from Baseline to Week 26 for AlC. Additional efficacy parameters include the mean change from Baseline in AlC at Week 6, Week 10, Week 14, Week 18, and Week 22. The proportion of subjects identified as responders will also be calculated. A responder is identified by any of the following four criteria: decrease from Baseline in A1C of 0.7%, decrease from Baseline in AlC of 0.5%, absolute AlC value of 7.0%, or mean decrease from Baseline in mean fasting blood glucose of 30 mg/dL at consecutive visits. The mean change from Baseline in all measured parameters will be calculated. Mean change from Baseline in the Homeostasis Model Assessment of Insulin Resistance (HOMA IR) as defined as insulin resistance = fasting serum insulin (µU/mL) x fasting plasma glucose (mmol/L)/22.5 will also be evaluated. Finally, the mean change from Baseline in the dose levels of each class of background oral hypoglycemic agents by treatment group will be analyzed.

All statistical tests will he one-sided and will be performed at the 0.050 significance level, unless otherwise specified. All statistical tests will he performed on both the ITT population and the per protocol population. Descriptive statistics, including mean, standard deviation, median, range, frequency distribution, and 95% one-sided confidence interval will be presented as appropriate.

## Claims

1. A transdermal hydroalcoholic gel composition for use **in the treatment of type-2 diabetes** in a subject in need thereof, wherein the transdermal hydroalcoholic gel composition comprises:
a. 0.01.% to 15% (w/w) of testosterone;
b. 0.01.% to 50% (w/w) penetration enhancing agent;
c. 0.01% to 50% (w/w) thickening agent;
d. 30% to 98% (w/w) lower alcohol; and
e. the balance water;
**wherein the serum testosterone concentration in the subject is at least 600 ng/dl to 1050 ng/dl after at least 30 days of daily administration of the composition.**

2. The transdermal hydroalcoholic gel composition according to claim 1 wherein the transdermal hydroalcoholic gel composition comprises:
a. 0.1% to 10% (w/w) of testosterone;
b. 0.1% to 5% (w/w) penetration enhancing agent;
c. 0.1% to 5% (w/w) thickening agent;
d. 30% to 98% (w/w) lower alcohol; and
e. the balance purified water.

3. The transdermal hydroalcoholic gel composition according to claim 1 or 2, wherein the composition comprises about 1% (w/w) of testosterone.

4. The transdermal hydroalcoholic gel composition according to any one of claims 1 to 3, wherein the penetration enhancing agent is selected from the group consisting of a C8-C22 fatty acid, a C8-C22 fatty alcohol, a lower alkyl ester of a C8-C22 fatty acid, a di(lower)alkyl ester of a C6-C22 diacid, a monoglyceride of a C8-C22 fatty acid, a tetrahydrofurfuryl alcohol polyethylene glycol ether, a polyethylene glycol, a propylene glycol, a 2-(2-ethoxyethoxy) ethanol, a diethylene glycol monomethyl ether, an alkylaryl ether of polyethylene oxide, a polyethylene oxide monomethyl ether, a polyethylene oxide dimethyl ether, a dimethyl sulfoxide, a glycerol, an ethyl
acetate, an acetoacetic ester, a N-alkylpyrrolidone, a terpene, isopropyl myristate and combinations thereof.

5. The transdermal hydroalcoholic gel composition of claim 4, wherein the penetration enhancing agent is isopropyl myristate.

6. The transdermal hydroalcoholic gel composition according to any one of claims I to 5, wherein the thickening agent is polyacrylic acid.

7. The transdermal hydroalcoholic gel composition according to any one of claims 1 to 6, wherein the transdermal hydroalcoholic gel composition comprises 45% to 90% ethanol or isopropanol.

8. The transdermal hydroalcoholic gel composition or claim 1, wherein the transdermal hydroalcoholic gel composition comprises:
a. about 1 % (w/w) testosterone;
b. about 0.9 % (w/w) polyacrylic acid;
c. about 0.5 % (w/w) isopropyl myristate;
d. about 67 % (w/w) ethanol; and
e. the balance purified water.

9. The trans dermal hydroalcoholic gel composition according to any one of claims 1 to 8, wherein the subject, has a pretreatment serum total testosterone concentration less than 300 ng/dL.

10. The transdermal hydroalcoholic gel composition according to any one of claims 1 to 9 wherein the transdermal hydroalcoholic gel composition is capable of releasing testosterone after applying the composition to the skin at a rate and duration that delivers at least 10 µg per day of testosterone to the blood serum of the subject.

11. The transdermal hydroalcoholic gel composition according to any one of claims 1 to 10, **wherein the serum testosterone concentration is at least 700 ng/dl serum to 1050 ng/dl serum.**

12. The transdermal hydroalcoholic gel composition according to any one of claims 1 to 11 wherein an amount of the transdermal hydroalcoholic gel composition is administered to an area of skin of the subject, which delivers a therapeutically-effective amount of testosterone to the blood serum of the subject.

13. The transdermal hydroalcoholic gel composition according to any one of claims I to 12, wherein the transdermal hydroalcoholic get composition is provided to the subject for daily administration in a 0.1 g to a 10 g dose.

14. The transdermal hydroalcoholic gel composition according to any one of claims 1 to 12, wherein the amount of the transdermal hydroalcoholic gel composition is a 5 to 10 g dose delivering 50 to 100 mg of testosterone to the skin.

15. The transdermal hydroalcoholic gel composition according to any one of claims 1 to 14, wherein said composition is administered once, twice, or three times daily for at least 7 days.

## Patentansprüche

1. Transdermale hydroalkoholische Gelzusammensetzung für die Verwendung bei der Behandlung von Typ-2-Diabetes bei einem Subjekt mit Bedarf daran, wobei die transdermale hydroalkoholische Gelzusammensetzung umfasst:
a. 0,01 % bis 15 % (Gew./Gew.) Testosteron;
b. 0,01 % bis 50 % (Gew./Gew.) penetrationsverstärkendes Mittel;
c. 0,01 % bis 50 % (Gew./Gew.) Verdickungsmittel;
d. 30 % bis 98 % (Gew./Gew.) Niederalkohol; und
e. als Rest Wasser;
wobei die Serum-Testosteronkonzentration in dem Subjekt nach wenigstens 30 Tagen mit täglicher Verabreichung der Zusammensetzung wenigstens 600 ng/dl bis 1050 ng/dl beträgt.

2. Transdermale hydroalkoholische Gelzusammensetzung gemäß Anspruch 1, wobei die transdermale hydroalkoholische Gelzusammensetzung umfasst:
a. 0,1 % bis 10 % (Gew./Gew.) Testosteron;
b. 0,1 % bis 5 % (Gew./Gew.) penetrationsverstärkendes Mittel;
c. 0,1 % bis 5 % (Gew./Gew.) Verdickungsmittel;
d. 30 % bis 98 % (Gew./Gew.) Niederalkohol; und
e. als Rest gereinigtes Wasser.

3. Transdermale hydroalkoholische Gelzusammensetzung gemäß Anspruch 1 oder 2, wobei die Zusammensetzung etwa 1 % (Gew./Gew.) Testosteron umfasst.

4. Transdermale hydroalkoholische Gelzusammensetzung gemäß einem der Ansprüche 1 bis 3, wobei das penetrationsverstärkende Mittel ausgewählt ist aus der Gruppe, bestehend aus einer C8-C22-Fettsäure, einem C8-C22-Fettalkohol, einem Niederalkylester einer C8-C22-Fettsäure, einem Di(nieder)alkylester einer C6-C22-Disäure, einem Monoglycerid einer C8-C22-Fettsäure, einem Tetrahydrofurfurylalkoholpolyethylenglycolether, einem Polyethylenglycol, einem Propylenglycol, einem 2-(2-Ethoxyethoxy)ethanol, einem Diethylenglycolmonomethylether, einem Alkylarylether von Polyethylenoxid, einem Polyethylenoxidmonomethylether, einem Polyethylenoxiddimethylether, einem Dimethylsulfoxid, einem Glycerol, einem Ethylacetat, einem Acetoessigsäureester, einem N-Alkylpyrrolidon, einem Terpen, Isopropylmyristat und Kombinationen davon.

5. Transdermale hydroalkoholische Gelzusammensetzung gemäß Anspruch 4, wobei das penetrationsverstärkende Mittel Isopropylmyristat ist.

6. Transdermale hydroalkoholische Gelzusammensetzung gemäß einem der Ansprüche 1 bis 5, wobei das Verdickungsmittel Polyacrylsäure ist.

7. Transdermale hydroalkoholische Gelzusammensetzung gemäß einem der Ansprüche 1 bis 6, wobei die transdermale hydroalkoholische Gelzusammensetzung 45 % bis 90 % Ethanol oder Isopropanol umfasst.

8. Transdermale hydroalkoholische Gelzusammensetzung gemäß Anspruch 1, wobei die transdermale hydroalkoholische Gelzusammensetzung umfasst:
a. etwa 1 % (Gew./Gew.) Testosteron;
b. etwa 0,9 % (Gew./Gew.) Polyacrylsäure;
c. etwa 0,5 % (Gew./Gew.) Isopropylmyristat;
d. etwa 67 % (Gew./Gew.) Ethanol; und
e. als Rest gereinigtes Wasser.

9. Transdermale hydroalkoholische Gelzusammensetzung gemäß einem der Ansprüche 1 bis 8, wobei das Subjekt eine Vorbehandlungs-Serum-Gesamtkonzentration von Testosteron von weniger als 300 ng/dl aufweist.

10. Transdermale hydroalkoholische Gelzusammensetzung gemäß einem der Ansprüche 1 bis 9, wobei die transdermale hydroalkoholische Gelzusammensetzung fähig ist, nach dem Applizieren der Zusammensetzung an die Haut Testosteron mit einer Rate und Dauer freizusetzen, die wenigstens 10 µg Testosteron pro Tag an das Blutserum des Subjekts liefert.

11. Transdermale hydroalkoholische Gelzusammensetzung gemäß einem der Ansprüche 1 bis 10, wobei die Testosteron-Serumkonzentration wenigstens 700 ng/dl Serum bis 1050 ng/dl Serum beträgt.

12. Transdermale hydroalkoholische Gelzusammensetzung gemäß einem der Ansprüche 1 bis 11, wobei eine Menge der transdermalen hydroalkoholischen Gelzusammensetzung an einen Bereich der Haut des Subjekts verabreicht wird, die eine therapeutisch wirksame Menge Testosteron an das Blutserum des Subjekts abgibt.

13. Transdermale hydroalkoholische Gelzusammensetzung gemäß einem der Ansprüche 1 bis 12, wobei die transdermale hydroalkoholische Gelzusammensetzung dem Subjekt zur täglichen Verabreichung mit einer Dosis von 0,1 g bis 10 g bereitgestellt wird.

14. Transdermale hydroalkoholische Gelzusammensetzung gemäß einem der Ansprüche 1 bis 12, wobei die Menge der transdermalen hydroalkoholischen Gelzusammensetzung eine Dosis von 5 bis 10 g ist, die 50 bis 100 mg Testosteron an die Haut abgibt.

15. Transdermale hydroalkoholische Gelzusammensetzung gemäß einem der Ansprüche 1 bis 14, wobei die Zusammensetzung einmal, zweimal oder dreimal täglich über wenigstens 7 Tage verabreicht wird.

## Revendications

1. Composition de gel hydroalcoolique transdermique pour une utilisation dans le traitement du diabète de type 2 chez un sujet en ayant besoin, dans laquelle la composition de gel hydroalcoolique transdermique comprend :
a. 0,01 % à 15 % (p/p) de testostérone ;
b. 0,01 % à 50 % (p/p) d'un agent favorisant la pénétration ;
c. 0,01 % à 50 % (p/p) d'un agent épaississant ;
d. 30 % à 98 % (p/p) d'un alcool inférieur ; et
e. le reste d'eau ;
dans laquelle la concentration sérique en testostérone chez le sujet est d'au moins 600 ng/dL à 1 050 ng/dL après au moins 30 jours d'administration quotidienne de la composition.

2. Composition de gel hydroalcoolique transdermique selon la revendication 1, dans laquelle la composition de gel hydroalcoolique transdermique comprend :
a. 0,1 % à 10 % (p/p) de testostérone ;
b. 0,1 % à 5 % (p/p) d'un agent favorisant la pénétration ;
c. 0,1 % à 5 % (p/p) d'un agent épaississant ;
d. 30 % à 98 % (p/p) d'un alcool inférieur ; et
e. le reste d'eau purifiée.

3. Composition de gel hydroalcoolique transdermique selon la revendication 1 ou 2, dans laquelle la composition comprend environ 1 % (p/p) de testostérone.

4. Composition de gel hydroalcoolique transdermique selon l'une quelconque des revendications 1 à 3, dans laquelle l'agent renforçant la pénétration est choisi dans le groupe constitué par un acide gras en C₈ à C₂₂, un alcool gras en C₈ à C₂₂, un ester d'alkyle inférieur d'un acide gras en C₈ à C₂₂, un diester d'alkyle (inférieur) d'un diacide en C₆ à C₂₂, un monoglycéride d'un acide gras en C₈ à C₂₂, un poly(éthylène glycol) éther d'alcool tétrahydrofurfurylique, un poly(éthylène glycol), un propylène glycol, un 2-(2-éthoxyéthoxy)éthanol, un diéthylène glycol monoéthyléther, un alkylaryléther de poly(oxyde d'éthylène), un monométhyléther de poly(oxyde d'éthylène), un diméthyléther de poly(oxyde d'éthylène), un diméthylsulfoxyde, un glycérol, un acétate d'éthyle, un ester acétoacétique, une N-alkylpyrrolidone, un terpène, le myristate d'isopropyle et une combinaison de ceux-ci.

5. Composition de gel hydroalcoolique transdermique selon la revendication 4, dans laquelle l'agent favorisant la pénétration est le myristate d'isopropyle.

6. Composition de gel hydroalcoolique transdermique selon l'une quelconque des revendications 1 à 5, dans laquelle l'agent épaississant est le poly(acide acrylique).

7. Composition de gel hydroalcoolique transdermique selon l'une quelconque des revendications 1 à 6, dans laquelle la composition de gel hydroalcoolique transdermique comprend 45 % à 90 % d'éthanol ou d'isopropanol.

8. Composition de gel hydroalcoolique transdermique selon la revendication 1, dans laquelle la composition de gel hydroalcoolique transdermique comprend :
a. environ 1 % (p/p) de testostérone ;
b. environ 0,9 % (p/p) de poly(acide acrylique) ;
c. environ 0,5 % (p/p) de myristate d'isopropyle ;
d. environ 67 % (p/p) d'éthanol ; et
e. le reste d'eau purifiée.

9. Composition de gel hydroalcoolique transdermique selon l'une quelconque des revendications 1 à 8, dans laquelle le sujet a une concentration sérique totale en testostérone de prétraitement inférieure à 300 ng/dL.

10. Composition de gel hydroalcoolique transdermique selon l'une quelconque des revendications 1 à 9, dans laquelle la composition de gel hydroalcoolique transdermique est capable de libérer de la testostérone après application de la composition sur la peau à un débit et pendant une durée d'administration d'au moins 10 µg par jour de testostérone dans le sérum sanguin du sujet.

11. Composition de gel hydroalcoolique transdermique selon l'une quelconque des revendications 1 à 10, dans laquelle la concentration sérique en testostérone est d'au moins 700 ng/dL de sérum à 1 050 ng/dL de sérum.

12. Composition de gel hydroalcoolique transdermique selon l'une quelconque des revendications 1 à 11, dans laquelle une quantité de la composition de gel hydroalcoolique transdermique est administrée à une région cutanée du sujet, qui délivre une quantité thérapeutiquement efficace de testostérone au sérum sanguin du suj et.

13. Composition de gel hydroalcoolique transdermique selon l'une quelconque des revendications 1 à 12, dans laquelle la composition de gel hydroalcoolique transdermique est fournie au sujet pour une administration quotidienne dans une dose de 0,1 g à 10 g.

14. Composition de gel hydroalcoolique transdermique selon l'une quelconque des revendications 1 à 12, dans laquelle la quantité de la composition de gel hydroalcoolique transdermique est une dose de 5 à 10 g délivrant 50 à 100 mg de testostérone à la peau.

15. Composition de gel hydroalcoolique transdermique selon l'une quelconque des revendications 1 à 14, dans laquelle ladite composition est administrée une fois, deux fois ou trois fois par jour pendant au moins 7 jours.
